# EUROPEAN PATENT APPLICATION

(11) **EP 4 512 302 A1**
(43) Date of publication of application: **26.02.2025**
(21) Application number: 24195223.3
(22) Date of filing: 19.08.2024
(51) Int. Cl.: A61B 1/00, A61B 1/018, A61B 1/05

(54) **INTERVENTIONAL MEDICAL DEVICE HAVING DATA COMMUNICATION**

(30) Priority: 22.08.2023 US 202318453347
(71) Applicant: Creganna Unlimited Company, Ballybrit, Galway (IE)
(72) Inventor: Mayevskiy, Yevgeniy, Wilsonville, 97070-9289 (US)
(74) Representative: Ashton, Gareth Mark

(57) **Abstract**

An interventional medical device (100) includes an insertion tube (120) extending between a proximal end (102) and a distal end (104). The distal end (104) configured to be inserted into internal tissues of a patient during a medical procedure. The insertion tube (120) has a dielectric waveguide (150) extending between the proximal end (102) and the distal end (104). The interventional medical device (100) includes a transmitter (230) coupled to the dielectric waveguide (150) at the distal end (104). The transmitter (230) configured to transmit a radio frequency signal along the dielectric waveguide (150) from the distal end (104) to the proximal end (102).

## Description

### BACKGROUND OF THE INVENTION

The subject matter herein relates generally to an interventional medical device.

Interventional medical devices, such as endoscopes, are used during medical procedures for performing medical procedures on a patient. An endoscope typically includes an elongated tube that may be inserted into the body of the patient. A video camera or a fiber optic lens is provided at the distal end for viewing the body tissues as the tube is moved through the patient's body. Various surgical tools may be inserted through the interior of the tube for performing surgical procedures. For example, some known endoscopes include needles for taking biopsies of tissue of a patient.

The image data from the video camera is transmitted through the elongated tube to an image processor. Some known interventional medical devices use electrical conductors, such as twisted wire pairs to transmit the data from the video camera to the image processor. However, as video quality increases, such as due to higher resolution cameras, higher frame rates, and increased color depth, the electrical conductors are approaching data transmission rate limits. Other known interventional medical devices use fiber optics to transmit signals from the video camera to the image processor. However, fiber optic systems are expensive.

A need remains for an interventional medical device having improved data communication.

### BRIEF DESCRIPTION OF THE INVENTION

In one embodiment, an interventional medical device is provided and includes an insertion tube extending between a proximal end and a distal end. The distal end configured to be inserted into internal tissues of a patient during a medical procedure. The insertion tube has a dielectric waveguide extending between the proximal end and the distal end. The interventional medical device includes a transmitter coupled to the dielectric waveguide at the distal end. The transmitter transmits a radio frequency signal along the dielectric waveguide from the distal end to the proximal end.

In another embodiment, an interventional medical device is provided and includes an insertion tube extending between a proximal end and a distal end. The distal end configured to be inserted into internal tissues of a patient during a medical procedure. The insertion tube has a dielectric waveguide extending between the proximal end and the distal end. The interventional medical device includes an imaging module at the proximal end of the insertion tube. The imaging module images the internal tissues of the patient during the medical procedure. The imaging device generates an image signal. The interventional medical device includes a transmitter coupled to the dielectric waveguide at the distal end. The transmitter transmits a radio frequency signal along the dielectric waveguide from the distal end to the proximal end. The radio frequency signal corresponds to the image signal from the imaging device.

In a further embodiment, an interventional medical device is provided and includes an insertion tube extending between a proximal end and a distal end. The distal end configured to be inserted into internal tissues of a patient during a medical procedure. The insertion tube has a dielectric waveguide extending between the proximal end and the distal end. The insertion tube including a lumen extending between the proximal end and the distal end. The interventional medical device includes a transmitter coupled to the dielectric waveguide at the distal end. The transmitter transmits a radio frequency signal along the dielectric waveguide from the distal end to the proximal end. The interventional medical device includes a tool received in the lumen. The tool configured to interact with the patient during the medical procedure.

### BRIEF DESCRIPTION OF THE DRAWINGS

Figure 1 is a perspective view of an interventional medical device in accordance with an exemplary embodiment.
Figure 2 is a schematic view of the interventional medical device showing the imaging system in accordance with an exemplary embodiment.
Figure 3 is a perspective view of the dielectric waveguide in accordance with an exemplary embodiment.
Figure 4 is a cross-sectional view of the dielectric waveguide in accordance with an exemplary embodiment.
Figure 5 is a cross-sectional view of the dielectric waveguide in accordance with an exemplary embodiment.
Figure 6 is a cross-sectional view of the dielectric waveguide in accordance with an exemplary embodiment.
Figure 7 is a cross-sectional view of the dielectric waveguide in accordance with an exemplary embodiment.
Figure 8 is a cross-sectional view of the insertion tube of the interventional medical device in accordance with an exemplary embodiment.
Figure 9 is a cross-sectional view of the insertion tube of the interventional medical device in accordance with an exemplary embodiment.

### DETAILED DESCRIPTION OF THE INVENTION

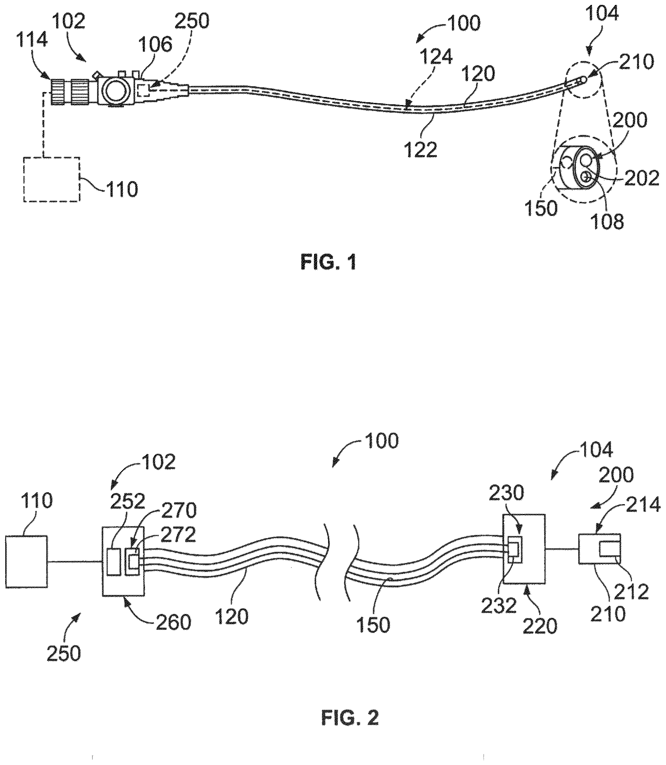

Figure 1 is a perspective view of an interventional medical device 100 in accordance with an exemplary embodiment. The interventional medical device 100 is used during a medical procedure for performing a medical procedure on a patient. In various embodiments, the interventional medical device 100 is an imaging device, such as an endoscope, a digital ultrasound device, a smart catheter, and the like. However, the interventional medical device 100 may be another type of medical device, such as a device including a MEMS sensor. In an exemplary embodiment, the interventional medical device 100 is configured to image internal tissues of a patient during a medical procedure. The interventional medical device 100 transmits image data through a tube or cable to a control unit at a base of the interventional medical device 100. The interventional medical device 100 may perform other tasks, such as drug delivery or tissue sampling or manipulation.

The interventional medical device 100 extends between a proximal end 102 and a distal end 104. An insertion tube 120 extends between the proximal end 102 and the distal end 104. The distal end 104 and the insertion tube 120 are configured to be inserted into the patient during the medical procedure. In an exemplary embodiment, a medical device is provided at the distal end 104 for interacting with the patient. The medical device may deploy a sensor, a tool, a camera, or another type of medical device. The tool may be a drug delivery tool, a needle, forceps, a balloon, an irrigation device, a sensor, or another type of tool. Controls for the medical device may extend through the insertion tube 120. Data from the medical device may be transmitted back to the operator at the proximal end through the insertion tube 120.

The interventional medical device 100 includes a handle 106 at the proximal end 102. The handle 106 may be used to actuate or manipulate the distal end 104 and/or one or more tools 108 at the distal end 104 for performing a medical procedure. For example, the distal end 104 may be steerable to position the tool 108 to perform the medical procedure, such as to biopsy damaged tissue or to deliver a drug. The tool 108 deployed via the working channel and/or controls for the tool 108 may extend within the insertion tube 120 between the handle 106 and the distal end 104. The tool 108 may be flush with the distal end 104 of the insertion tube 120. However, the tool 108 may be recessed within the insertion tube 120 at the distal end 104 or may extend from the distal end 104. The handle 106 may be rotated and/or translated to actuate or manipulate the distal end 104, such as using pull wires, springs, or other types of actuation elements extending between the handle 106 and the distal end 104. In various embodiments, electrical wires may extend between the proximal end 102 and the distal end 104 to power/control the tool 108 or other components of the interventional medical device 100, such as being routed in one or more internal channels of the insertion tube. The electrical wires may be used for data transmission.

In an exemplary embodiment, the interventional medical device 100 includes a viewing device 110. The viewing device 110 may include an eyepiece coupled to the handle 106. In various embodiments, the viewing device 110 may include a display, such as a computer monitor showing an image. Images or other data may be displayed or viewable at the exterior of the interventional medical device 100, such as at a remote computer monitor, at an attached display or dial, or at another location.

In an exemplary embodiment, the interventional medical device 100 includes an imaging system 200 for imaging the internal tissues of the patient. The imaging system 200 includes an imaging module 210 at the distal end 104 and a control unit 250 at the proximal end 102. The imaging module 210 may define a tip or imaging end of the medical device 100 (for example, endoscope). The insertion tube 120 connects the imaging module 210 and the control unit 250. Data is transmitted between the imaging module 210 and the control unit 250 through the insertion tube 120. In an exemplary embodiment, the insertion tube 120 includes a dielectric waveguide 150 for data transmission between the imaging module 210 and the control unit 250.

In an exemplary embodiment, the imaging system 200 includes a light transmitter 202 at the distal end 104 for illuminating the internal tissues. The light transmitter 202 may include one or more optical fibers and/or light pipes routed between the proximal end 102 and the distal end 104. The light transmitter 202 may include a lens or LED sources at the distal end 104 that emits the light.

The insertion tube 120 extending between the proximal end 102 and the distal end 104. The insertion tube 120 is flexible. The distal end 104 of the insertion tube 120 is configured to be inserted into the patient and manipulated through the internal tissues of the body off the patient to a desired location. The insertion tube 120 includes a sleeve 122 at an exterior of the insertion tube 120. Other components are routed through the hollow interior of the sleeve 122. The sleeve 122 may be manufactured from medical grade material, such as a stainless steel material, a nitinol material, or another suitable material. The sleeve 122 may be coated, such as with a PTFE spray or jacket. The sleeve 122 may include multiple sections. Portions of the sleeve 122 may be rigid. Portions of the sleeve 122 may be flexible to allow manipulation of the insertion tube 120 within the body of the patient.

In an exemplary embodiment, various components of the interventional medical device 100 are housed within the insertion tube 120 and extend between the handle 106 and the distal end 104. The sleeve 122 includes one or more internal channels, including at least one working channel 124, that houses the components of the interventional medical device 100. For example, the tool 108 may be routed through the working channel 124 to the distal end 104. The light transmitter 202 may be routed through the working channel 124 or another internal channel to the distal end 104. The working channel 124 may be separate from or isolated from other internal channels. The internal channels may be open lumens extend between the opposite ends of the insertion tube 120. In an exemplary embodiment, the dielectric waveguide 150 is routed through the working channel 124 to the imaging module 210 at the distal end 104. Other components may be routed through the internal channel(s), such as air tubes, water tubes, electrical wires, and the like.

Figure 2 is a schematic view of the interventional medical device 100 showing the imaging system 200 in accordance with an exemplary embodiment. The interventional medical device 100 includes the insertion tube 120 extending between the proximal end 102 and the distal end 104. The imaging module 210 is provided at the distal end 104 and is used for imaging the internal tissues of the patient during the medical procedure. The control unit 250 is provided at the proximal end 102 and is used to control the imaging module 210. In an exemplary embodiment, the dielectric waveguide 150 extends between the proximal end 102 and the distal end 104 for data transmission between the imaging module 210 and the control unit 250.

The imaging module 210 includes an imaging device, such as a camera 212, and an integrated circuit 220 operably coupled to the camera 212. The camera 212 may include a sensor 214, such as a complementary metal-oxide-semiconductor (CMOS) sensor. The camera 212 may include a lens. The integrated circuit 220 receives signals from the camera 212. The camera 212 generates image signals transmitted to the integrated circuit 220. The image signals are digital signals. The integrated circuit 220 supports the digitization of the imaging signals. The imaging module 210 and/or the integrated circuit 220 may include a processor, such as an image processor, to digitize the images from the camera 212. In various embodiments, the camera 212 is a high-resolution color camera for imaging the tissues. The camera 212 may be a high resolution camera such as 4K or 8K UHDTV video camera. The camera 212 may have a high frame rate, such as 60 frames per second or more. Other types of imaging devices may be used in alternative embodiments, such as an ultrasound probe. The integrated circuit 220 may include a circuit board and one or more electrical components mounted to the circuit board.

In an exemplary embodiment, the imaging module 210 includes a transmitter 230 coupled to or integrated with the integrated circuit 220. The transmitter 230 transmits data signals from the imaging module 210 to the control unit 250. For example, the transmitter 230 may transmit signals from the camera 212 to the control unit 250. Optionally, the transmitter 230 may transmit signals from other components, such as a sensor 214 at the distal end 104, in addition to the signals from the camera 212. The transmitter 230 is a wireless transmitter. In an exemplary embodiment, the transmitter 230 includes a transmit antenna 232 coupled to the integrated circuit 220. The transmit antenna 232 transmits radio frequency signals. For example, the transmit antenna 232 converts the image signals to the radio frequency signals. The radio frequency signal corresponds to the image signal from the imaging module. For example, the RF signal may be generated from or based on the image signal. The image signal may be modulated into the transmitted radio frequency signal. The image signal may be digitized and serialized to the RF signal. The transmit antenna 232 may be configured to support gigabit range data transmission, such as between 3Gbps and 30Gbps, or more. The transmit antenna 232 may be a microwave antenna. In an exemplary embodiment, the radio frequency signals are at a frequency of at least 30 GHz. For example, the radio frequency signals may be in an extremely high frequency (EHF) range, such as between 30 GHz and 300 GHz. In various embodiments, the radio frequency signals may be at approximately 150GHz. The transmitter 230 is coupled to the dielectric waveguide 150 to transmit the radio frequency signals along the dielectric waveguide 150. In various embodiments, the transmitter 230 may be a transceiver configured to both transmit and receive radio frequency signals.

The control unit 250 includes a processor 252 and an integrated circuit 260 operably coupled to the processor 252. The integrated circuit 260 receives the radio frequency signals from the dielectric waveguide 150. In an exemplary embodiment, the control unit 250 includes a receiver 270 coupled to or integrated with the integrated circuit 260. The receiver 270 receives the radio frequency signals from the dielectric waveguide 150 and converts the radio frequency signals to digital signals. For example, the receiver 270 includes a receive antenna 272 coupled to the dielectric waveguide 150 that receives the radio frequency signals from the dielectric waveguide 150 and converts the radio frequency signals to digital signals. The processor 252 processes the digital signals. The digital signals may be sent to the viewing device 110 for viewing by the operator. In various embodiments, the receiver 270 may be a transceiver configured to both transmit and receive radio frequency signals.

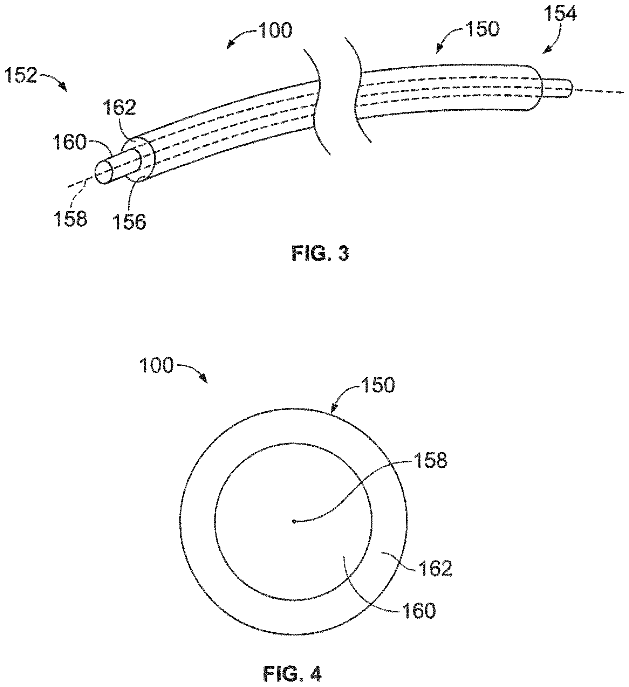

Figure 3 is a perspective view of the dielectric waveguide 150 in accordance with an exemplary embodiment. Figure 4 is a cross-sectional view of the dielectric waveguide 150 in accordance with an exemplary embodiment. The dielectric waveguide 150 extends between a proximal end 152 and a distal end 154. The distal end 154 is configured to be coupled to the imaging module 210, such as to the transmitter 230 (shown in Figure 2). The proximal end 152 is configured to be coupled to the control unit 250, such as to the receiver 270 (shown in Figure 2).

The dielectric waveguide 150 is flexible. The dielectric waveguide 150 has a small diameter, such as approximately 1.0 mm. The dielectric waveguide 150 includes one or more dielectric layers 156. The dielectric layers 156 may be concentric about a central axis 158 of the dielectric waveguide 150. The radio frequency signals are transmitted along at least one of the dielectric layers 156. In an exemplary embodiment, the dielectric waveguide 150 includes a central core 160 and a cladding 162 surrounding the core 160. The radio frequency signals are transmitted along the central core 160. The dielectric waveguide may include other layers in alternative embodiments.

The central core 160 is formed of a solid dielectric material configured to conduct radio waves at millimeter wave frequencies and above. The central core 160 may be manufactured from a first dielectric material, such as a polymer material including, but not limited to, vinylidene fluoride, tetrafluoroethylene and/or hexafluorpropylene. The central core 160 may be manufactured by an extrusion process. In various embodiments, the central core 160 is filled with the polymer material. In other various embodiments, the central core 160 may be hollow, such as forming a lumen that may receive other components or tools of the interventional medical device 100.

The cladding 162 has a lower relative permittivity or a lower dielectric constant than the core 160 and confines the radio waves using total internal reflection. The cladding 162 may have a different dielectric constant than the core 160. The cladding 162 contains the transmission waves within core 160. The cladding 162 may be constructed with materials that reflect waves back into core 160. For example, the cladding 162 may be formed of materials that have dielectric constants that are different than the dielectric constant of the material forming the core 160. The cladding 162 may have a dielectric constant that is less than core 160. In various embodiments, the cladding 162 is a foamed cladding, such as being formed from a plastic foam such as, but not limited to, polystyrene foam. In other various embodiments, the cladding 162 may be an extruded plastic material formed around the core 160.

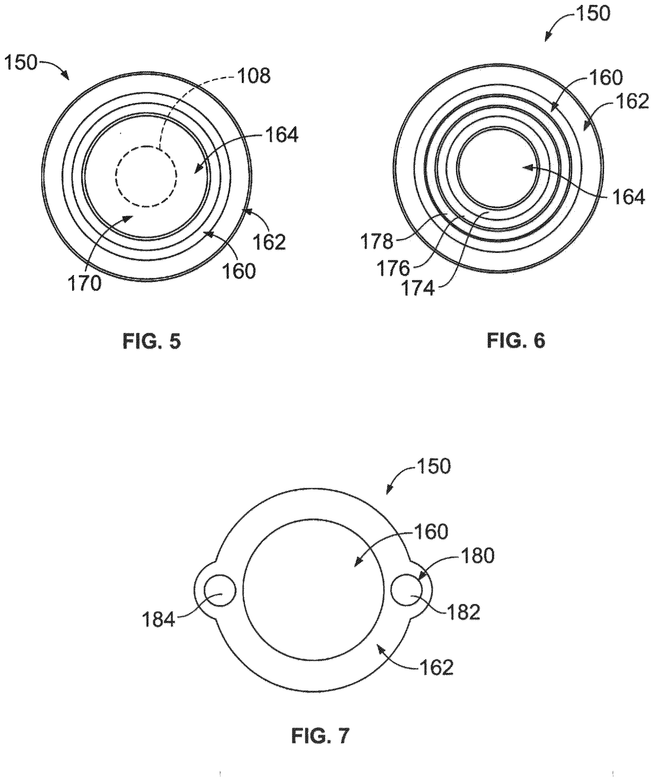

Figure 5 is a cross-sectional view of the dielectric waveguide 150 in accordance with an exemplary embodiment. The dielectric waveguide 150 includes the core 160 and the cladding 162. In the illustrated embodiment, the core 160 is hollow having an air core 164 along the central axis 158. In various embodiments, the air core 164 forms a lumen 170 configured to receive other components, such as the tool 108 (shown in Figure 1) and/or control wires for the tool 108. For example, the inner part of the dielectric waveguide 150 can serve as a working lumen. The dielectric waveguide 150 may include a metal shield layer between the inner dielectric layer (for example, the core 160) and the outer dielectric layer (for example, the cladding 162). In alternative embodiments, the metal layer may be provided around the outside of the cladding 162. An outer jacket or sleeve may be provided around the outside of the dielectric waveguide 150 in other embodiments. In alternative embodiments, the working channel deploying the tool 108 or other components may be routed through separate lumens/internal channels from the dielectric waveguide 150 rather than being routed through the core 160 of the dielectric waveguide 150.

Figure 6 is a cross-sectional view of the dielectric waveguide 150 in accordance with an exemplary embodiment. The dielectric waveguide 150 includes the core 160 and the cladding 162. The core 160 includes the air core 164. In the illustrated embodiment, the core 160 includes multiple concentric dielectric layers, such as a first layer 174, a second layer 176, and a third layer 178. The second layer 176 is located between the first and third layers 174, 178. The layers 174, 176, 178 may be extruded layers. The second layer 176 is formed from a different dielectric material having a different dielectric constant than the first layer 174 and the third layer 178. In various embodiments, the first layer 176 is formed from a different dielectric material than the third layer 178. However, in other various embodiments, the first layer 176 is formed from the same dielectric material as the third layer 178. The first layer 174 and/or the second layer 176 and/or the third layer 178 is configured to transmit or propagate the radio frequency signals. The core 160 may include greater or fewer layers in alternative embodiments. The cladding 162 may include multiple layers in alternative embodiments.

Figure 7 is a cross-sectional view of the dielectric waveguide 150 in accordance with an exemplary embodiment. The dielectric waveguide 150 includes the core 160 and the cladding 162. In the illustrated embodiment, the core 160 is a solid core. However, in alternative embodiments, the core 160 may be hollow having an air core.

In an exemplary embodiment, one or more conductors 180 are arranged in the dielectric waveguide 150. The conductors 180 may be electrical conductors or optical conductors. The conductors 180 extend between the proximal end 102 and the distal end 104. The electrical conductors 180 may be electrically connected to components at the proximal end and/or the distal end, such as the imaging module 210, the camera 212, the transmit antenna 232, the sensor 214, or other components. In an exemplary embodiment, the electrical conductors 180 include a power conductor or wire 182 and a ground conductor or wire 184. Other types of conductors may be provided in other embodiments, such as low speed data signal conductors.

In an exemplary embodiment, the electrical conductors 180 pass through channels in the cladding 162. The electrical conductors 180 may be provided on opposite sides of the dielectric waveguide 150. However, other locations are possible in alternative embodiments. For example, the electrical conductors 180 may be arranged in the core 160 or in the outer jacket or sleeve (not shown) in alternative embodiments.

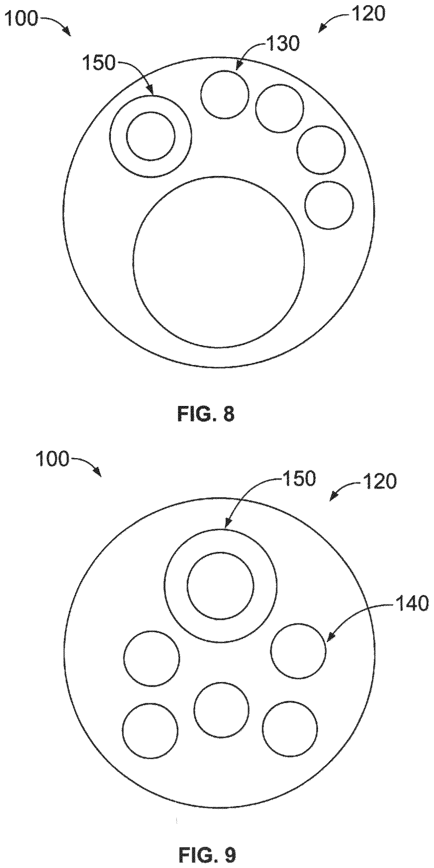

Figure 8 is a cross-sectional view of the insertion tube 120 of the interventional medical device 100 in accordance with an exemplary embodiment. The interventional medical device 100 may be an endoscope shaft having the working channel 124, the dielectric waveguide 150 and a plurality of metal wires or interconnects 130 in the insertion tube 120. The metal wires or interconnects 130 may be used for control, power, and/or data transfer. The insertion tube 120 may include other components within the inner portion of the insertion tube 120.

Figure 9 is a cross-sectional view of the insertion tube 120 of the interventional medical device 100 in accordance with an exemplary embodiment. The interventional medical device 100 may be an ultrasound cable assembly having the dielectric waveguide 150 and a plurality of metal wires or interconnects 140 in the insertion tube 120. The metal wires or interconnects 140 may be used for control, power, and/or data transfer. The insertion tube 120 may include other components within the inner portion of the insertion tube 120.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the invention without departing from its scope. Dimensions, types of materials, orientations of the various components, and the number and positions of the various components described herein are intended to define parameters of certain embodiments, and are by no means limiting and are merely exemplary embodiments. Many other embodiments and modifications within the scope of the claims will be apparent to those of skill in the art upon reviewing the above description. The scope of the invention should, therefore, be determined with reference to the appended claims.

## Claims

1. An interventional medical device (100) comprising:
an insertion tube (120) extending between a proximal end (102) and a distal end (104), the distal end (104) configured to be inserted into internal tissues of a patient during a medical procedure, the insertion tube (120) having a dielectric waveguide (150) extending between the proximal end (102) and the distal end (104); and
a transmitter (230) coupled to the dielectric waveguide (150) at the distal end (104), the transmitter (230) configured to transmit a radio frequency signal along the dielectric waveguide (150) from the distal end (104) to the proximal end (102).

2. The interventional medical device (100) of claim 1, further comprising a receiver (270) coupled to the dielectric waveguide (150) at the proximal end (102), the receiver (270) configured to receive the radio frequency signal from the dielectric waveguide (150).

3. The interventional medical device (100) of claim 1 or 2, wherein the insertion tube (120) includes a lumen (170) concentric with the dielectric waveguide (150), a tool (108) being received in the lumen, the tool (108) configured to interact with the patient during a medical procedure.

4. The interventional medical device (100) of any preceding claim, further comprising an imaging module (210) at the distal end (104) and a control unit (250) at the proximal end (102) operably coupled to the imaging module (210), the transmitter (230) allowing data communication from the imaging module (210) to the control unit (250) via the dielectric waveguide (150).

5. The interventional medical device (100) of claim 4, wherein the imaging module (210) includes a camera (212) and an integrated circuit (220) receiving signals from the camera (212), the transmitter (230) including a transmit antenna (232) coupled to the integrated circuit (220) configured to transmit the radio frequency signal, the control unit (250) including an integrated circuit (260) having a receive antenna (272) configured to receive the radio frequency signal from the dielectric waveguide (150) and convert the radio frequency signal to a digital signal, the control unit (250) including a data processor (252) configured to process the digital signal.

6. The interventional medical device (100) of any preceding claim, wherein the transmitter (230) includes an integrated circuit (220) configured to receive a digital signal, the transmitter (230) including an antenna (232) coupled to the integrated circuit (220) and configured to convert the digital signal to the radio frequency signal.

7. The interventional medical device (100) of any preceding claim, further comprising a sensor (214) at the distal end (104), the transmitter (230) configured to receive signals from the sensor (214) and convert the signals to the radio frequency signal for transmission to the proximal end (102) through the dielectric waveguide (150).

8. The interventional medical device (100) of any preceding claim, wherein the dielectric waveguide (150) includes a central core (160) and a cladding (162) surrounding the core (160), the central core (160) being configured to transmit the radio frequency signal.

9. The interventional medical device (100) of any preceding claim, wherein the insertion tube (120) includes an electrical conductor (180) extending between the proximal end (102) and the distal end (104).

10. The interventional medical device (100) of any preceding claim, wherein the insertion tube (120) includes a power wire (182) and a ground wire (184) extending between the proximal end (102) and the distal end (104).

11. The interventional medical device (100) of any preceding claim, wherein the dielectric waveguide (150) includes multiple concentric dielectric layers (156, 174, 176, 178).

12. The interventional medical device (100) of any preceding claim, wherein the dielectric waveguide (150) includes an inner dielectric layer (160), an outer dielectric layer (162), and a metal shield layer between the inner dielectric layer (160) and the outer dielectric layer (162).

13. The interventional medical device (100) of any preceding claim, wherein the dielectric waveguide (150) is flexible.

14. The interventional medical device (100) of any preceding claim, wherein the radio frequency signal is at a frequency of at least 30 GHz.

15. The interventional medical device (100) of any preceding claim, wherein the radio frequency signal is between 30 GHz and 300 GHz.
